# EUROPEAN PATENT APPLICATION

(11) **EP 1 179 520 A1**
(43) Date of publication of application: **13.02.2002**
(21) Application number: 00117199.0
(22) Date of filing: 11.08.2000
(51) Int. Cl.: C07C 29/36, C07D 317/20, C07C 45/59, C07C 45/62, C07C 45/29, C07C 29/40, C07C 45/69

(54) **Process for the preparation of phytone and novel intermediates thereof**

(71) Applicant: AVENTIS ANIMAL NUTRITION S.A., 92164 Antony Cedex (FR)
(72) Inventor: Ancel, Jean-Erick, 69230 Saint-Genis-Laval (FR); Couture, Karine, 69350 La Mulatiere (FR)
(74) Representative: Mérigeault, Shona

(57) **Abstract**

Novel intermediate compounds which can be used in the preparation of phytone and Vitamin E and a process for the preparation thereof. A process for the preparation of phytone from the intermediate compounds is also claimed.

## Description

The present invention relates to a process for the preparation of phytone and in particular a process for the preparation of phytone from 3,7, dimethyl 1,6, octadiene, generally referred to as citronellene

Phytone is an intermediate used in the preparation of Vitamin E. US Patent No. 3867408 discloses the preparation of phytone from novel ketal compounds. The phytone, so produced, is then used to prepare Vitamin E. US Patent No. 4168271 discloses a method for the preparation of Vitamin E from intermediate compounds which are isomers of dehydrotocopherol.

We have now developed an alternative process for the preparation of certain compounds that may be used in the synthesis of phytone, thus providing a novel route in the synthesis of phytone and thus a novel route in the synthesis of vitamin E.

Accordingly, the present invention provides a process for the preparation of compound of general formula (I) 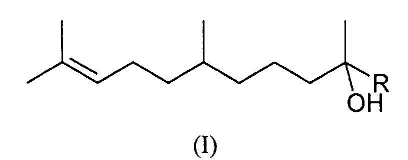 wherein R represents hydrogen or a hydrocarbon optionally containing an oxygenated functional group, by reacting a compound of general formula (II) 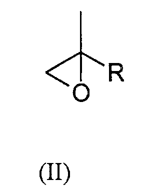 wherein R is as hereinbefore defined with a magnesium halide salt of 3,7, dimethyl 1,6, octadiene, in the presence of a copper (I) compound and an organic solvent.

For the purposes of the present invention, 3,7, dimethyl 1,6, octadiene is hereafter referred to as citronellene.

Certain compounds of general formula (I) are novel and as such also form another aspect of this invention.

The process of the present invention involves the catalytic reaction between a compound of general formula (II) and a magnesium halide salt of citronellene. Considering the compound of formula (II), R is hydrogen or a hydrocarbon which may contain an oxygenated functional group. Where R is a hydrocarbon, the hydrocarbon may be a linear hydrocarbon and may be saturated or unsaturated. Where R is a hydrocarbon containing an oxygenated functional group, R may be a ketal, ketone, aldehyde or an ether. Preferably, R comprises an oxygenated functional group. The preferred compounds according to general formula (II) are propylene oxide and the following compound, known as 2-methyl 2-propyl 1,3-dioxolane:

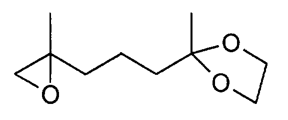

The magnesium halide salt of citronellene may be any suitable halide, for example chloride or bromide. The preferred halide is chloride.

The magnesium salt of citronellene may be obtained by adding a magnesium compound to citronellene in the presence of a catalyst. Suitable catalysts include titanium compounds such as TiCl₄ and TiCp₂Cl₂. Suitably, the magnesium compound may be a magnesium alkyl chloride having at least two carbons, for example ethyl, propyl or isopropyl magnesium chloride. Suitably the magnesium salt of citronellene is prepared in-situ during the process of the present invention.

The mole ratio of compound of general formula (II) to the magnesium halide salt is suitably from 0.5 to 2, preferably from 0.8 to 1.2 molar equivalents.

The process of the present invention is carried out in the presence of a copper(I) compound. Suitable copper(I) compounds include copper(I) halides, for example chloride, bromide and iodide; copper(I) acetate and copper(I) triflate. Preferably, the copper(I) compound is a copper(I) halide, especially copper(I) chloride. The amount of copper(I) compound present in the reaction is suitably from 0.005 to 0.1, preferably from 0.01 to 0.1 molar equivalent.

The process is also carried out in the presence of an organic solvent. Suitable solvents include aromatic solvents, for example toluene and xylene; ethers such as tetrahydrofuran, diethyl ether and isopropyl ether. The preferred solvent is tetrahydrofuran. The amount of solvent present in the reaction system is suitably from 3 to 100, preferably from 5 to 20 mass equivalents.

The process may be carried out at a temperature of from -80 to +150°C, preferably from -50 to +25°C and under atmospheric or elevated pressure. Preferably, the reaction is carried out under atmospheric pressure. The process is also suitably carried out under an inert atmosphere. Suitable inert gases include argon and nitrogen.

Certain compound of general formula (I) are novel and as such form another aspect of the present invention. In particular compounds of general formula (I) wherein R is H or R is 2 methyl 2 propyl 1,3, dioxolanne are novel compounds.

We have found that when R of general formula (I) is a hydrocarbon containing an oxygenated functional, these compounds can be used in the synthesis of phytone and thus, according to another aspect of the present invention there is provided a process for the preparation of phytone which comprises (a) a first step hydrolysing a compound according to general formula (I) hereinbefore defined wherein R is a hydrocarbon containing an oxygenated functional group; and (b) a second step of hydrogenating the hydrolysis product produced in said first step.

In particular,we have found that phytone can be accessed through this prepartion method, where R is a ketal, especially 2-methyl 2-propyl-1,3-dioxolane.

The first step, namely the hydrolysis step, of the process is suitably carried using an acid catalyst such as sulphonic acid, or hydrogen chloride. The catalyst may be present in an amount of from 0.001 to 0.5 molar equivalents, preferably between 0.05 and 0.1 molar equivalents, compared to the compound of formula (I).

The hydrolysis is also suitably carried out in the presence of an organic solvent such as toluene or an ether, for example diethyl ether or tetrahydrofuran. The temperature of the reaction may be between -50 and +150°C, preferably between 20 and 100°C.

The product of the hydrolysis step is then hydrogenated. The hydrogenation is suitably carried out in the presence of hydrogen gas and in the presence of a metal or metal salt. Suitable metals and metal salts include Raney nickel (a nickel/aluminium alloy) optionally in the presence of iron, mangenese, cobalt, copper, zinc or chromium; zinc in the presence of acetic acid; stannous chloride; and molybdenum (III) salts. The reaction may also be carried out in the presence of palladium or platinum which may be supported on an suitable inert support such as charcoal. The hydrogenation is preferably carried out in the presence of palladium on an inert support such as on charcoal. . The amount of metal or metal salt employed is generally from 0.01 to 3 molar equivalents, preferably from 0.05 to 2 molar equivalents.

The hydrogenation step is generally conducted in a solvent which may be selected from alcohols such as methanol or ethanol; ethers; and aromatic hydrocarbons . The preferred solvent is an alcohol, especially methanol and ethanol.

The reaction temperature in the hydrogenation step is generally from 20°C to 150°C, preferably from 20°C to 90°C and under a gas a pressure of 1 to 50 bars, preferably 5 to 10 bars is generally used.

The hydrolysis and hydrogenation may be carried out as two separate steps or combined as one step in the reaction system.

The process for the preparation of phytone is suitably carried out for a period of time from 30 minutes to 24 hours, preferably from 30 minutes to 6 hours under the aforementioned reaction conditions in order to facilitate complete reaction of the reaction compounds.

Phytone may also be prepared from compound of general formula (I) as hereinbefore defined where R is hydrogen, namely propylene oxide, but is this case, the syntheses route generally requires additional steps. When R is H, compound of general formula (I) gives access to Phytone by a known procedure which comprises the following steps (1) oxidizing the 6,10-dimethyl-1-decen-9-ol-2 to obtain the corresponding ketone; (2) reacting the ketone thus formed with a vinyl magnesium salt to form 3,7,11-trimethyl 1,10-dodecadiene-ol; (3) reacting the allylic alcohol with methyl aceto acetate in presence of a Lewis acid catalyst to obtain methyl acetoacetate to give 6,10,14-trimethyl 5,13 -pentadecadiene -2-one; and (4) hydrogenating the unsaturated C₁₈ ketone thus formed, to obtain phytone.

The phytone produced according to the aforementioned processes may be used in the synthesis of Vitamin E. Thus, according to another aspect of the present invention there is provided Vitamin E obtained from phytone prepared as hereinbefore described.

The present invention will now be illustrated with reference to the following examples:

### Example 1:

Step (1) - The first step of the reaction scheme as detailed below was carried out under an inert atmosphere using argon and under the reaction conditions as detailed in Table 1.

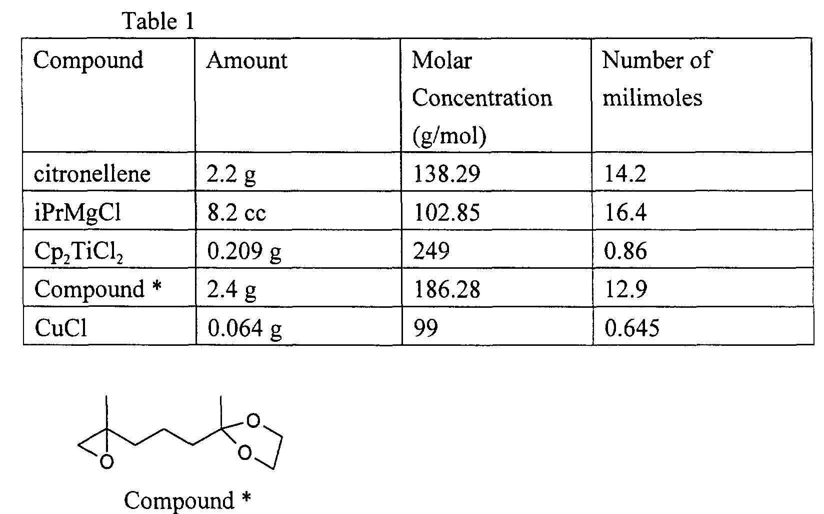

Dichloro titanocene was introduced, under an inert atmosphere, into a flask equipped with two necks. The isopropyl magnesium chloride salt, in 2 mole/litre in tetrahydrofuran (THF), was then added at ambient temperature, followed by citranellene. The reaction mixture immediately turned black in colour and slight rise in temperature was observed. The flask was then placed in an oil bath at 100°C. The reaction was left to proceed for 4 hours under these conditions and then the contents of the flask were rapidly cooled to 0°C. In a second flask equipped with two necks, a solution containing the epoxy acetal, the copper chloride salt and anhydrous THF was prepared by mixing the components for 15 minutes at ambient temperature. This solution was added drop by drop to the magnesium compound at 0°C over a period of 15 minutes. The resulting mixture was then left for 2 hours 20 minutes at 0°C.

A solution of saturated aqueous sodium carbonate was then added to the product mixture. The resulting product was then extracted three times with diethyl ether. The organic phases were collected, dried using magnesium sulphate, filtered and concentrated under vacuum. 4.37 g of product was obtained.

Steps (2) and (3) - The second and third steps of the reaction scheme as detailed below was carried out under the reaction conditions as detailed in Table 2.

**Table 2**

| Compound | Amount | Molar Concentration (g/mol) | Number of milimoles |
|---|---|---|---|
| Product of step 1 | 0.933 g | 326.58 | 2.46 |
| Toluene | 30 ml | - | - |
| APTS.H₂O | 0.01 g | 190.22 | 0.053 |
| HCl | 20 micro litres | - | 0.23 |
| THF | 3 ml | - | - |
| Ethanol | 15 ml | - | - |
| Pd-black | 0.117 g | 106.42 | 0.055 |

The reactant, toluene and the APTS were placed in a 25 ml round bottom flask and left to react for 3 hours under reflux of toluene. 10 ml of a saturated aqueous solution of sodium carbonate was then added and the resulting product extracted three times with ether.

The reaction mixture was added to 1 ml of THF and 0.05 ml of hydrochloric acid. The solution was then left for a further hour at ambient temperature. A little magnesium sulphate was then added, the contents of the flask stirred for 5 minutes to dry the organic phase and then filtered and concentrated under vacuum. The product obtained (0.37 g) was immediately hydrogenated.

The product was immediately placed in a glass vial containing the palladium, and ethanol under an argon atmosphere. The vial was placed in an autoclave. The autoclave was sealed shut and purged with hydrogen. The hydrogen pressure was fixed at 5 ±0.2 bars and the contents agitated. The hydrogenation reaction was continued for 6 hours at ambient temperature. The autoclave was then degassed and contents poured into a little column containing a filtration celite. The column was washed with ethanol and the filtrate concentrated. A weight of 0.677 g of phytone was obtained. The yield of product was 77% with 95% purity.

### Example 2:

Step (1) - The first step of the reaction scheme, as detailed below, was carried out as in Example 1 under the reaction conditions as detailed in Table 3

**Table 3**

| Compound | Amount | Molar Concentration (g/mol) | Number of milimoles |
|---|---|---|---|
| citronellene | 10 g | 138.28 | 64.4 |
| iPrMgCl | 35.4 ml | 102.85 | 70.8 |
| Cp₂TiCl₂ | 0.98 g | 249 | |
| Propylene oxide | 4 ml | 58.08 | 57.9 |
| CuCl | 0.088 g | 99 | 0.89 |

Dichlorotitanocene was introduced, under an inert atmosphere, into a flask equipped with two necks. The isopropyl magnesium chloride salt (2mole/litre) in tetrahydrofuran (THF), was then added at ambient temperature, followed by citronellene. The reaction mixture immediately turned black in colour and slight rise in temperature was observed. The flask was then placed in an oil bath at 100°C. The reaction was left for 4 hours under these conditions and then rapidly cooled to 0°C. The copper chloride salt, in powdered form, was then added using a glove box. The propylene oxide was then quickly added over 2 minutes using a syringe. The resulting mixture was then left for 2 hours at 0°C. A solution of aqueous ammonium chloride was then added and the resulting product extracted with diethyl ether. The organic phase was separated, dried with magnesium sulphate, filtered and concentrated under vacuum. The product, 8,12 g of 6,10-dimethyl-undec-9-en2-ol, was obtained with a yield of 71%.

Step 2: - The product of the previous step was oxidised under the following conditions:

**Table 4**

| Compound | Amount | Molar Concentration (g/mol) | Number of milimoles |
|---|---|---|---|
| C13 alcohol | 0.23 g | 198.39 | 1.12 |
| CH₂Cl₂ anhydrous | 37 ml | - | - |
| Molecular sieve 4 A | 0.1 g | - | - |
| CaCO₃ | 0.465 | 100.09 | 4.67 |
| PCC | 0.825 g | 215.56 | 3.83 |

The alcohol was placed in a flask equipped with three necks. The flask was then purged with argon. The dichloromethane, molecular sieve, and the calcium carbonate were then added successively. The mixture was stirred at ambient temperature for 15 minutes. The PCC was then added. The resulting mixture was then stirred vigorously for 90 minutes wherein the colour changed to black. The product was filtered on a celite pad and the pad was washed with diethyl ether; the filtrate was washed with an aqueous solution of saturated sodium chloride and then extracted five times with 15ml of diethyl ether. The organic phase was separated, dried with magnesium sulphate, filtered and concentrated under vacuum. 0.2 g of product was obtained, giving a yield of 79%.

Step 3: - The product of the previous step underwent condensation with vinyl magnesium chloride under the following reaction conditions:

**Table 5**

| Compound | Amount | Molar Concentration (g/mol) | Number of milimoles |
|---|---|---|---|
| Compound obtained in previous step | 0.466 g | 196.39 | 2.37 |
| Vinyl magnesium chloride 15% weight in THF | 1.83 ml | 86.81 | 3.085 |
| Anhydrous THF | 1 ml + 1 ml | - | - |

The vinyl magnesium chloride, in tetrahydrofuran (THF), was placed in a flask equipped with two necks which had been previously purged with argon. The resulting solution was heated to 35°C ± 2°C. The ketone prepared in step (2) was dissolved in 1ml of THF and then added to the solution drop by drop over 90 minutes using a syringe. The syringe was rinsed with 1ml of THF, which was introduced rapidly into the reactant solution. The resulting mixture was left for 20 minutes at a temperature of 35°C. Aqueous sulphuric acid (pH 1) was added at ambient temperature and the mixture then extracted three times with 20ml of diethyl ether. The organic phase was separated, dried with magnesium sulphate, filtered and concentrated under vacuum. 0.444 g of product, giving a yield of 80% and 95% purity, was obtained.

Step 4: - The product of the previous step underwent condensation under the following reaction conditions:

**Table 6**

| Compound | Amount | Molar Concentration (g/mol) | Number of milimoles |
|---|---|---|---|
| Compound obtained in the previous step | 1.18g | 224.43 | 5.26 |
| Methyl acetoacetate | 0.735 ml | 116.12 | 6.84 |
| AlO(iPr)₃ | 0.054 g | 204.25 | 0.263 |

The product obtained in step (3) and tris isopropylate aluminium were placed in a flask equipped with two necks. The resulting mixture was left with stirring at ambient temperature for 30 minutes under a argon atmosphere. The reactants were then heated to 90°C. The methyl acetoacetate was then added and the temperature then increased to 150°C over 1 hour 40 minutes. The temperature was maintained at 155°C for 5 hours. The temperature was then reduced to ambient and the contents of the flask left stirring under argon. 2ml of water containing 0.1 ml of 96% sulphuric acid was then added to the reactants. The mixture was left for a further 20 minutes at ambient temperature. Two phases became apparent and the organic phase was collected by decantation whilst the aqueous phase was extracted with diethyl ether. The organic phase was separated, dried with magnesium sulphate, filtered and concentrated under vacuum. 1.34g of product, giving a yield of 74% and greater than 95% purity, was obtained.

Step 5: - Hydrogenation of 6,10,14-trimethyl 5,13,pentadecadiene-2-one (DHP)

**Table 7**

| Compound | Amount | Molar Concentration (g/mol) | Number of milimoles |
|---|---|---|---|
| DHP | 0.583 g | 264.5 | 2.2 |
| ethanol | 10 ml | - | - |
| Pd-black (5%) | 0.0935 g | 106.42 | 0.044 |

The DHP, the palladium and ethanol were placed in a glass vial which had been purged with argon. The vial was placed in an autoclave. The autoclave was sealed shut and purged with hydrogen. The hydrogen pressure was fixed at 5 ±0.2 bars the contents agitated. The hydrogenation reaction was continued for 6 hours at ambient temperature. The autoclave was then degassed and contents poured into a little column containing celite . The column was washed with ethanol and the filtrate concentrated. A weight of 0.544 g of phytone, giving a yield of 81% and a purity greater than 95%, was obtained.

### REACTION SCHEMES RELATING TO EXAMPLES 1 AND 2

### Example 1

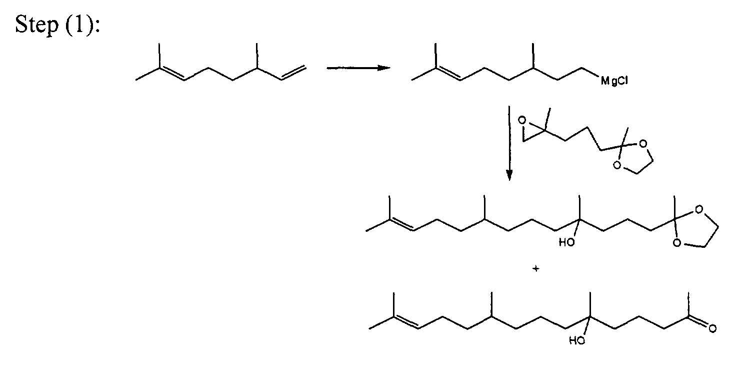

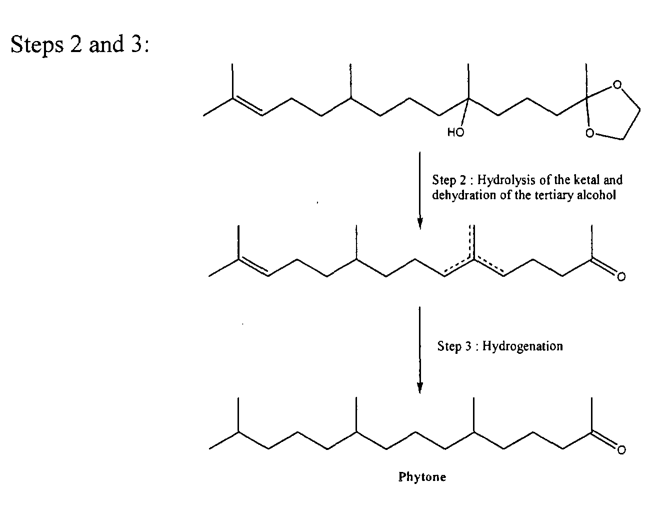

### Example 2

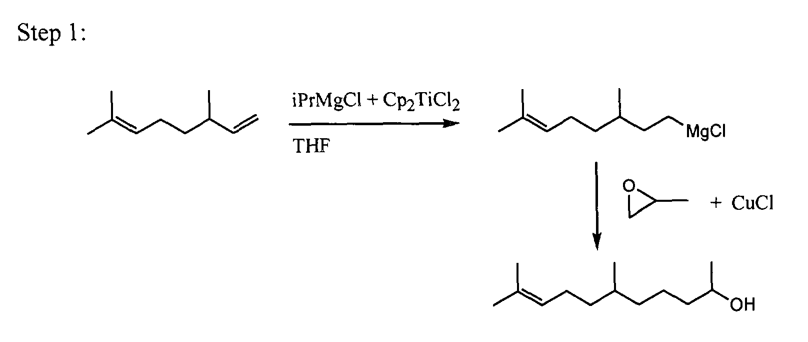

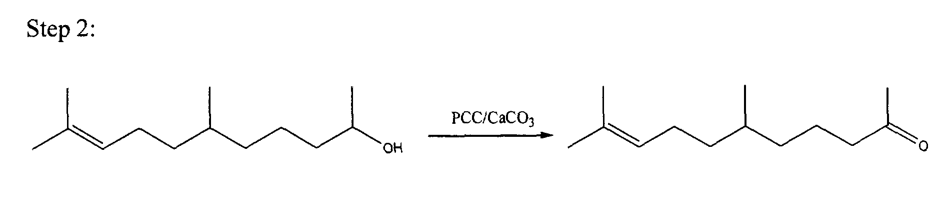

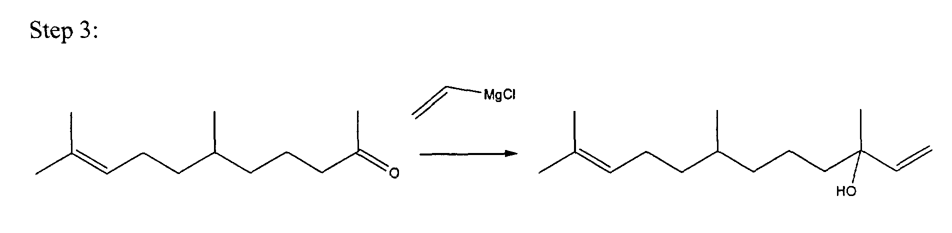

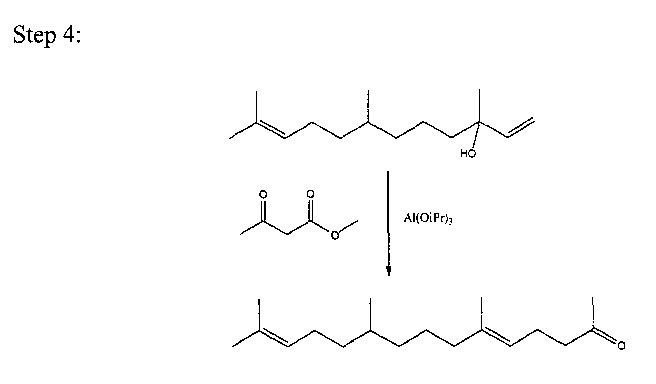

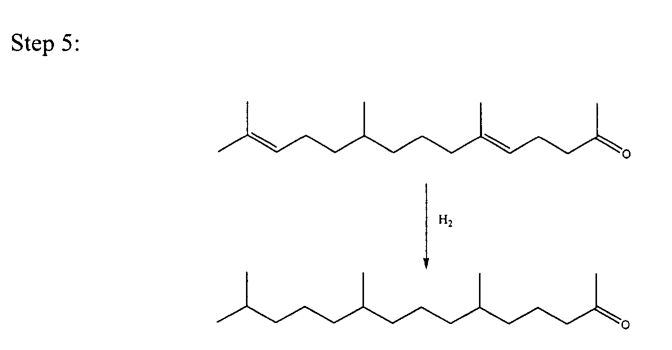

## Claims

1. A process for the preparation of a compound of general formula (I) 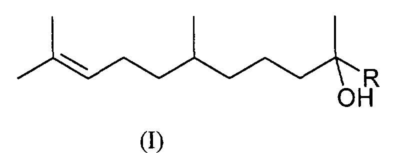 wherein R represents hydrogen or a hydrocarbon optionally containing an oxygenated functional group, by reacting a compound of general formula (II) 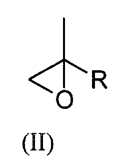 wherein R is as hereinbefore defined, with a magnesium halide salt of 3,7, dimethyl 1,6, octadiene, in the presence of a copper(I) compound and an organic solvent.

2. A process as claimed in claim 1 wherein R is a hydrocarbon containing an oxygenated functional group.

3. A process as claimed in claim 2 wherein R is a ketal.

4. A process as claimed in any one of the preceding claims wherein the magnesium halide salt of 3,7, dimethyl 1,6, octadiene is the chloride salt.

5. A process as claimed in any one of the preceding claims wherein the copper(I) salt is copper(I) halide, copper(I) acetate or copper(I) triflate.

6. A process as claimed in claim 5 wherein the copper(I) salt is a copper(I) halide, preferably copper(I) chloride.

7. A process as claimed in any one of the preceding claims wherein the organic solvent is selected from toluene, xylene, tetrahydrofuran, diethyl ether and isopropyl ether.

8. Novel compounds according to general formula (I) as defined in claim 1 wherein R is hydrogen and 2methyl-2-propyl 1,3, dioxolane.

9. A process for the preparation of phytone which comprises (a) a first step of hydrolysing a compound of general formula (I) where R is a hydrocarbon containing an oxygenated functional group, and (b) a second step of hydrogenating the hydrolysis product of the first step.

10. A process as claimed in claim 9 wherein R is a ketal, preferably 2-methyl 2-propyl-1,3- dioxolane.

11. A process as claimed in claim 9 or claim 10 wherein the first step is carried out in the presence of an acid catalyst selected from sulphonic acid and hydrogen chloride.

12. A process as claimed in any one of claims 9 to 11 wherein the first step is carried out in the presence of an organic solvent selected from an organic hyrdocarbon and an ether.

13. A process as claimed in any one of claims 9 to 12 wherein the second step is carried out in the presence of hydrogen and a metal or metal salt selected from palladium or platinum, Raney nickel optionally in the presence of iron, mangenese, cobalt, copper, zinc or chromium; zinc in the presence of acetic acid; stannous chloride; and molybdenum (III) salts.

14. A process as claimed in claim 13 wherein the catalyst is palladium supported on charcoal.

15. A process for the prepartion of phytone which comprises (a) a first step of preparing a compound according to of general formula (I) as defined in claim 1 wherein R is hydrogen; (b) a second step of oxidizing the 6,10-dimethyl-1-decen-9-ol-2 produced in the first step to obtain the corresponding ketone; (c) reacting the ketone thus formed with a vinyl magnesium salt to form an allylic alcohol 3,7,11-trimethyl 1,10-dodecadiene-ol; (d) reacting the allylic alcohol with methyl aceto acetate in presence of a Lewis acid catalyst to obtain to give 6,10,14-trimethyl 5,13 -pentadecadiene -2-one; and (4) hydrogenating the unsaturated C₁₈ ketone thus formed.

16. Vitamin E obtained from phytone **characterised in that** the phytone is prepared according to a process as claimed in any one of claims 9 to 15.
